# FASCICULE DE BREVET EUROPEEN

(11) **EP 3 669 902 B1**
(45) Date de publication et mention de la délivrance du brevet: **28.04.2021**
(21) Numéro de dépôt: 18306770.1
(22) Date de dépôt: 20.12.2018
(51) Int. Cl.: A61L 31/02

(54) **DISPOSITIF DE REGENERATION OSSEUSE GUIDEE ET PROCEDE DE FABRICATION**
VORRICHTUNG ZUR GEFÜHRTEN KNOCHENWIEDERHERSTELLUNG, UND HERSTELLUNGSVERFAHREN
DEVICE FOR GUIDED BONE REGENERATION AND MANUFACTURING METHOD

(43) Date de publication de la demande: 24.06.2020
(73) Titulaire: Badaoui, Ralphe, 75002 Paris (FR); Nammour, Joseph, 75004 Paris (FR)
(72) Inventeur: Badaoui, Ralphe, 75002 Paris (FR); Nammour, Joseph, 75004 Paris (FR)
(74) Mandataire: Novagraaf Technologies

(56) Documents cités:
- Anderud: "ON GUIDED BONE REGENER ATION USING CERAMIC MEMBRANES DOCTORAL DISSERTATION IN ODONTOLOGY", , 1 janvier 2016 (2016-01-01), XP55597214, Extrait de l'Internet: URL:http://hdl.handle.net/2043/20132 [extrait le 2019-06-17]
- JONAS ANDERUD ET AL: "Guided bone augmentation using ceramic space-maintaining devices: the impact of chemistry", CLINICAL, COSMETIC AND INVESTIGATIONAL DENTISTRY, 1 mars 2015 (2015-03-01), page 45, XP55597230, DOI: 10.2147/CCIDE.S78589

## Description

### Domaine technique

La présente invention se rapporte à un dispositif de régénération osseuse guidée, destiné à la reconstruction d'un défaut osseux buccal, ainsi qu'à un procédé de fabrication d'un tel dispositif.

Ainsi, la présente invention trouve des applications dans le domaine médical, et plus particulièrement le domaine de la chirurgie dentaire.

Dans la description ci-dessous, les références entre crochets ([]) renvoient à la liste des références présentée à la fin du texte.

### Etat de la technique

Afin de régénérer des tissus détruits et/ou disparus en raison de perte osseuse, il existe plusieurs techniques d'augmentation osseuse, comme les greffes d'apposition, les greffes d'interposition, l'expansion, la distraction, les greffes de sinus ou encore la régénération osseuse guidée (ROG).

La ROG a pour objet de recréer de l'os afin de pouvoir placer et/ou stabiliser un implant dentaire, ou de recréer une crête pour une prothèse inamovible. Classiquement, une membrane est placée entre l'os et la gencive, pendant 3 à 6 mois, avant de pouvoir utiliser l'implant ou réaliser la prothèse inamovible. La membrane utilisée forme une barrière physique, permettant d'une part d'empêcher la colonisation du défaut osseux par les tissus mous conjonctifs et épithéliaux, et d'autre part limitant l'accès à l'espace cicatriciel aux seule cellules à pouvoir ostéogénique. Ces membranes ont ainsi un triple rôle : prévenir la prolifération des cellules à partir de la muqueuse de recouvrement et favoriser la migration des cellules issues des espaces médullaires dans le caillot habitant le site, stabiliser la greffe osseuse et le caillot et stabiliser le comblement par biomatériau.

Dans cette optique, plusieurs types de membranes, résorbables ou non résorbables, sont utilisés, comme par exemple les membranes en polytétrafluoroéthylène (PTFE) ou en collagène résorbable. Les caractéristiques des membranes non résorbables sont surtout l'inertie biologique, la flexibilité, la stabilité chimique et la micro-porosité asymétrique. En revanche, elles nécessitent une fixation par des vis et une deuxième intervention pour la dépose.

Au contraire, les membranes résorbables présentent l'avantage de ne pas devoir être retirées après la régénération osseuse, ce qui évite une potentielle irritation des tissus pouvant se produire lors du retrait, si la membrane s'est liée au tissu environnant pendant la phase de cicatrisation. Toutefois, les membranes résorbables ont pour inconvénient une possible interférence entre la résorption/cicatrisation et la régénération osseuse, ainsi que la nécessité d'un matériau supportant la membrane.

Il existe donc un réel besoin d'un dispositif palliant ces défauts, en particulier permettant d'obtenir une modélisation optimale de la reconstruction, ainsi qu'un mimétisme et une symétrie des structures osseuse.

Jonas Anderud et. al.,"Guided bone augmentation using ceramic spacemaintaining devices: the impact of chemistry", Clinical, Cosmetic and Investigational Dentistry, 1 mars 2015, p.45 décrit des écarteurs sous formes de plaques ou dômes rigides de dioxyde de zircornium qui permettent une régénération osseuse guidée pour la reconstruction d'un défaut osseux buccal.

### Description de l'invention

Les Demandeurs ont conçu un dispositif répondant à ces objectifs.

En effet, le dispositif de l'invention permet une augmentation osseuse, tant verticale qu'horizontale, permettant de combler le défaut osseux, une modélisation optimale de la reconstruction, ainsi qu'un mimétisme et une symétrie des structures osseuse.

Ainsi, un premier objet de l'invention se rapporte à un dispositif de régénération osseuse guidée, destiné à la reconstruction d'un défaut osseux. IL est composé de dioxyde de zirconium (ZrO₂) et ayant une forme recouvrant ledit défaut osseux buccal, ledit dispositif comprenant au moins une fenêtre aménagée dans la paroi de celui-ci et destinée à l'insertion et à la condensation d'un biomatériau.

Avantageusement, le dispositif de l'invention a une forme tridimensionnelle recouvrant au moins en partie, et de préférence totalement, le défaut osseux. Ainsi, à l'issue de la reconstruction osseuse, le volume reconstruit peut être substantiellement identique à celui dû à la perte osseuse traitée, voire supérieur.

Afin d'obtenir une forme tridimensionnelle recouvrant la forme du défaut osseux à traiter, toute technique de modélisation du volume et/ou de la quantité osseuse à reconstruire peut être utilisée. Il peut s'agir d'une technique d'imagerie dento-maxillaire du défaut osseux, comme par exemple la tomographie volumique à faisceaux coniques (également appelée Cône beam). Avantageusement, le défaut osseux peut être schématisé numériquement sur un logiciel adapté pour quantifier la substance osseuse à régénérer, comme par exemple les logiciel MIMICS ou 3-matic, cette liste n'étant pas limitative.

Le dispositif de l'invention est ainsi fabriqué en fonction de la forme modélisée, de manière à mimer la planification 3D, par toute technique adaptée et connue de l'homme du métier, comme par exemple par moulage, par usinage ou par impression 3D. Eventuellement, à partir du schéma, une couche supplémentaire de 1 mm d'épaisseur peut être rajoutée à la représentation 3D, avant de construire le dispositif de l'invention, pour avantageusement pallier une formation épithéliale superficielle qui peut se produire dans certaines régénérations.

Avantageusement, le dispositif de l'invention possède une forme appropriée lui permettant de recouvrir tout ou une partie du défaut osseux et de permettre la reconstruction de tout le volume osseux perdu, si nécessaire. Avantageusement, quelle que soit la forme choisie, le dispositif ménage un espace creux, lors de son installation, entre la paroi de celui-ci et l'os résiduel, de manière à permettre l'introduction d'un biomatériau à l'intérieur du dispositif de l'invention. Toute forme permettant d'atteindre cet objectif peut être utilisée par l'homme du métier. Il peut s'agir par exemple d'une forme choisie parmi une coquille, une plaque rigide, coque, et un filet (également appelé mesh).

Ainsi qu'énoncé précédemment, le dispositif de l'invention est composé de dioxyde de zirconium (également appelé zyrcone ou oxyde de zyrconium). La teneur du dispositif en dioxyde de zirconium peut être par exemple comprise entre 88% et 96%, par exemple 88%, ou 89%, ou 90% ou 91% ou 92% ou 93% ou 94% ou 95% ou 96%. Avantageusement, au moins un autre constituant classiquement présent dans ce type de céramique peut être présent, comme par exemple l'oxyde d'yttrium, l'oxyde d'hafnium et/ou l'oxyde d'aluminium. Par exemple, l'oxyde d'yttrium peut être présent à hauteur de 4 à 6%, l'oxyde d'hafnium peut être présent à hauteur de 1 - 5% et l'oxyde d'aluminium peut être présent à hauteur de 0 à 1%. Il peut s'agir par exemple d'une composition commerciale, comme par exemple Zfx™ Zircone (Zfx GmbH, Allemagne). Avantageusement, le dioxyde de zirconium est utilisé dans le dispositif de l'invention pour ses propriétés physiques connues, comme sa résistance à la rupture et à la fissuration, son haut degré de biocompatibilté, sa neutralité, et le fait qu'il induise très peu d'inflammation. Avantageusement, le zircone constitue une barrière cellulaire et tissulaire. De plus, il est avantageusement non ostéointegrable, et son retrait est facile. Grâce aux caractéristiques du dispositif de l'invention, le greffon est avantageusement stabilisé et compressé, et montre un niveau de résorption quasiment nul.

Avantageusement, le dispositif de l'invention peut comprendre au moins une perforation pour la stabilisation du dispositif, chaque perforation étant destinée à recevoir une vis d'ostéosynthèse. A ce titre, la ou les perforation(s) est/sont réalisée(s) sur la paroi vestibulaire du dispositif. Le nombre de perforations peut être déterminé selon des critères classiques appliqués dans ce domaine, par exemple en fonction de la taille du défaut osseux. Il peut s'agir à ce titre d'un nombre de perforations compris entre 1 et 4, voire entre 1 et 20, voire plus, en fonction des besoins et du volume à reconstruire.

Le dispositif de l'invention comprend au moins une fenêtre aménagée dans sa paroi, destinée à l'insertion d'un biomatériau et sa condensation. Le biomatériau, une fois inséré entre l'os et la paroi du dispositif de l'invention, c'est-à-dire dans l'espace à reconstruire, va permettre une ostéoinduction et ainsi reconstruire l'os souhaité. Tout type de biomatériau adapté à la reconstruction osseuse peut être utilisé dans ce cadre, comme tout type de substitut osseux choisi parmi les substituts osseux allogéniques, xénogeniques, autogéniques et synthétiques, cette liste n'étant pas limitative. La taille, la forme et le nombre de fenêtres peut être choisi par l'homme du métier en fonction notamment de la taille du dispositif de l'invention, ainsi que de la nature des défauts osseux à traiter. Par exemple, la fenêtre peut être de forme carrée, ronde, ovale ou rectangulaire. Elle peut par exemple être inscrite dans un rectangle de1 à 20 mm de haut et de 1 à 30 mm de large ou plus. La fenêtre peut être dessinée selon toute technique adaptée connue de l'homme du métier, par exemple au moyen d'un planificateur 3D.

Le dispositif peut avoir une épaisseur adaptée à sa fonction, c'est-à-dire permettre une protection du biomatériau le temps nécessaire à la reconstruction osseuse. A ce titre, l'homme du métier peut aisément déterminer l'épaisseur adaptée en fonction des cas. Par exemple, l'épaisseur du dispositif de l'invention peut être comprise entre 0,4 mm et 2,5 mm, par exemple 0,4mm, 0,5mm, 0,6 mm ou 0,7 mm ou 0,8 mm ou 0,9 mm ou 1,0 mm ou 1,1 mm ou 1,2 mm ou 1,3 mm ou 1,4 mm ou 1,5 mm ou 1,6 mm ou 1,7 mm ou 1,8 mm, ou 1,9 mm, ou 2,0 mm, ou 2,1 mm, ou 2,2 mm, ou 2,3 mm, ou 2,4 mm, ou 2,5 mm.

Avantageusement, le dispositif de l'invention est facile à retirer une fois le temps de pose nécessaire à la reconstruction osseuse atteint. En effet, celui-ci recouvrant, par l'extérieur, le volume à reconstruire, il suffit de l'enlever une fois la ou les vis retirées.

Avantageusement, le dispositif de l'invention peut comprendre en outre, de manière optionnelle, des perforations au niveau crestal, pour permettre l'insertion d'implants dentaires lors de l'étape chirurgicale de la régénération osseuse guidée.

Un autre objet de l'invention se rapporte à un procédé de fabrication d'un dispositif de l'invention tel que défini précédemment. Le procédé mis en œuvre peut comprendre notamment une étape de construction dudit dispositif en fonction d'une représentation 3D du défaut osseux obtenue par une technique d'imagerie dento-maxillaire, par exemple au moyen d'une technique de tomographie volumique à faisceaux coniques (Cône beam).

Avantageusement, et comme indiqué précédemment, la représentation 3D du défaut osseux peut être schématisée numériquement sur un logiciel adapté pour quantifier la substance osseuse à régénérer.

Eventuellement, la schématisation numérique permet d'obtenir un schéma, à partir duquel le dispositif de l'invention va être fabriqué de manière à recouvrir la représentation 3D du défaut osseux. Eventuellement, il est possible d'ajouter une couche de 1mm d'épaisseur supplémentaire, de manière à augmenter le volume à couvrir et obtenir un volume reconstruit supérieur, et à palier a une formation d'une couche épithéliale superficielle.

Un autre objet de l'invention se rapporte à un procédé de régénération osseuse comprenant les étapes de :
- mise en place d'un dispositif selon l'invention au niveau d'un défaut osseux d'un patient,
- introduction de biomatériau dans le dispositif,
- pose du dispositif pendant un temps suffisant à la condensation du biomatériau, et
- retrait du dispositif de l'invention.

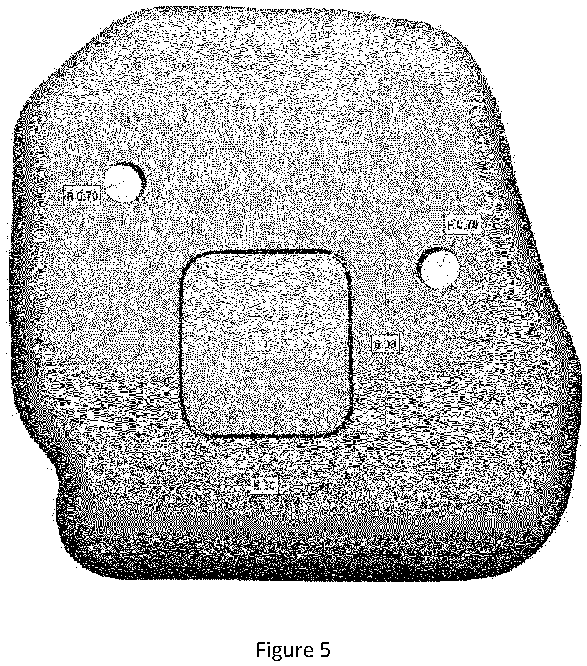

D'autres avantages pourront encore apparaître à l'homme du métier à la lecture des exemples ci-dessous, illustrés par les figures annexées, donnés à titre illustratif. Seule la figure 5 représente un dispositif selon l'invention.

### Brève description des figures

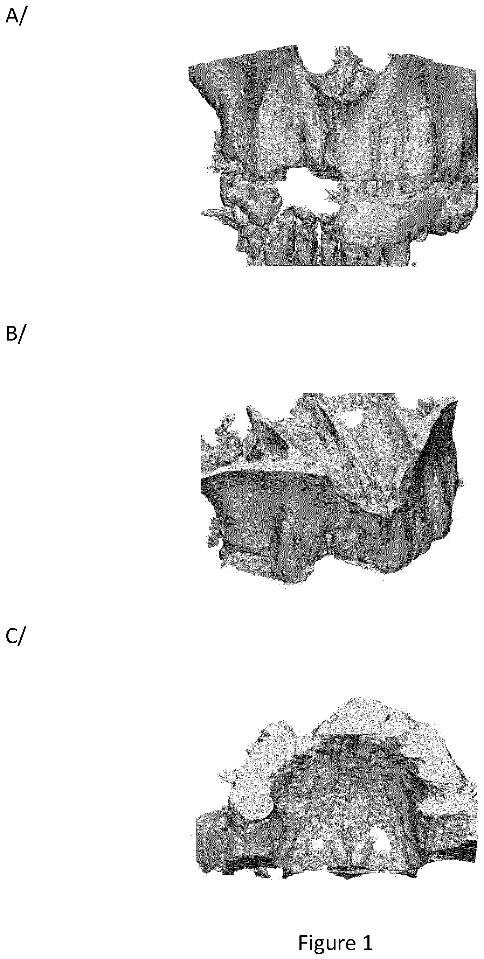

- La figure 1 représente une reconstruction volumique osseuse schématisée en 3 dimensions. A : vue de face du volume à reconstruire. B : vue du dessus du volume à reconstruire. C : vue du dessous du volume à reconstruire.

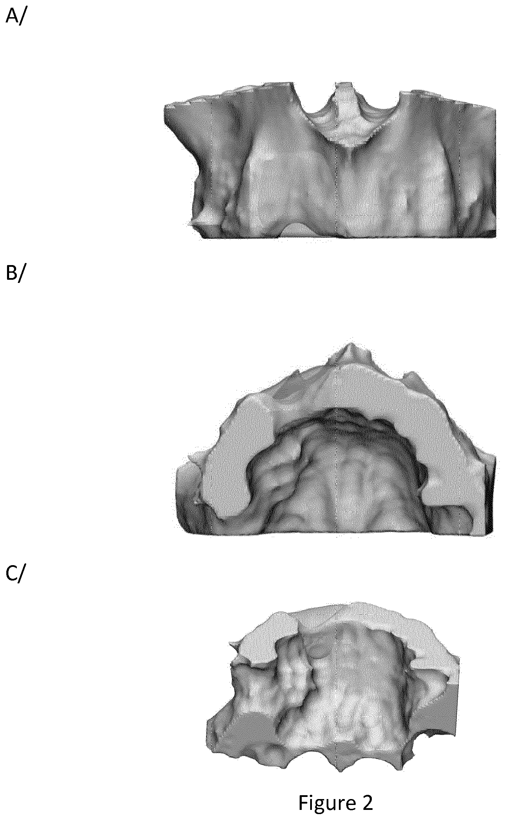

- La figure 2 représente une vue du volume osseux à reconstruire après nettoyage et définition du volume de comblement par effet miroir. A : vue de face du volume à reconstruire. B : vue du dessus du volume à reconstruire. C : vue du dessous du volume à reconstruire.

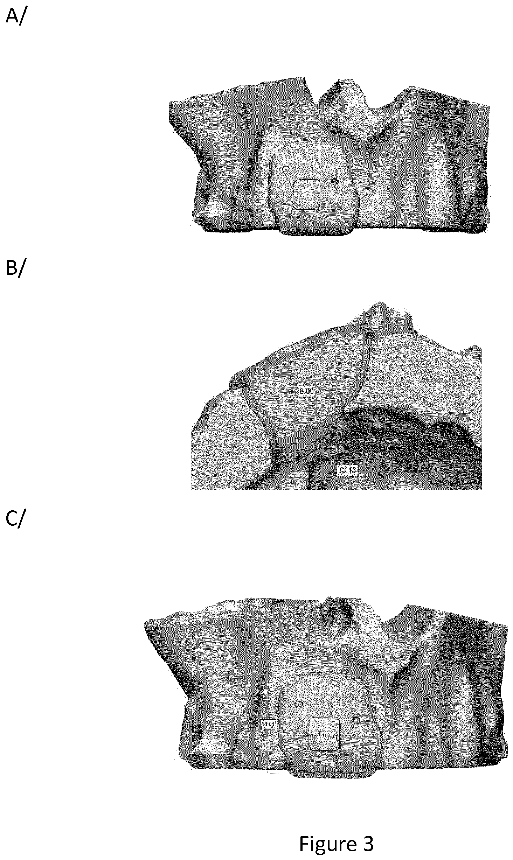

- La figure 3 représente une vue d'une coque d'épaisseur 0,8mm avec un décalage de 1mm au-dessus du volume à combler. A : vue de face du volume à reconstruire. B : vue du dessus du volume à reconstruire. C : vue de face du volume à reconstruire.

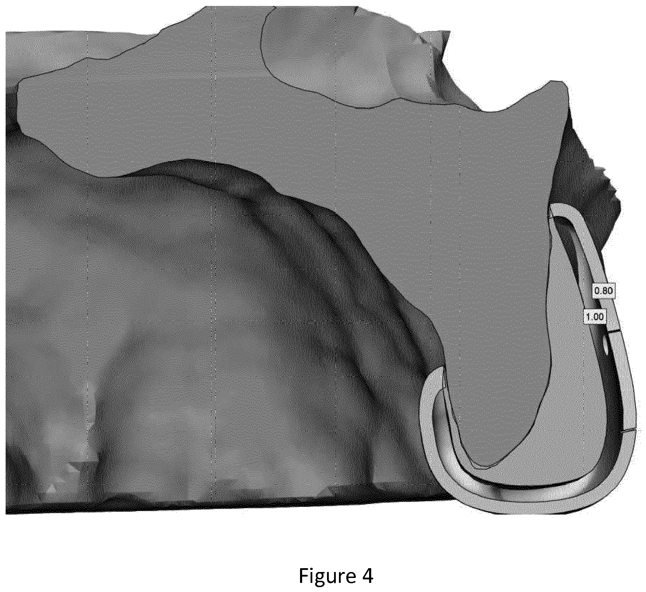

- La figure 4 représente une vue en coupe d'une coquille recouvrant le volume osseux à reconstruire.
- La figure 5 représente une vue de face d'une coquille, comportant une fenêtre de 6 mm de haut et de 5,5 mm de large, et deux trous de fixation d'un diamètre de 1,4 mm chacun.

### EXEMPLES (non conformes à l'invention)

### Exemple 1 : préparation d'un dispositif destiné à une régénération osseuse guidée en forme de coquille

On réalise une modélisation du volume de la quantité osseuse à reconstruire au moyen d'une tomographie volumique à faisceaux coniques (Cône beam).

Une représentation 3D de la reconstruction du défaut osseux est schématisée numériquement sur le logiciel MIMICS/3-matic, permettant de quantifier la substance osseuse à régénérer.

Grâce à ce schéma, une couche supplémentaire « optionnelle » de 1mm d'épaisseur est rajoutée à la représentation 3D, puis une coquille de 0,6 à 1,8 mm d'épaisseur est conçue de manière à couvrir la planification 3D évoquée.

Des perforations sont dessinées afin de stabiliser la future coquille de zircone grâce à des vis d'ostéosynthèse.

Une fois le schéma de la coquille validé, la coquille est imprimée en zircone au moyen d'une imprimante 3D.

### Exemple 2 : Exemple de mise en œuvre du dispositif de régénération osseuse guidée

Des médicaments préopératoires ont été prescrits au patient: acide amoxicilline clavulanique (2 g par jour), prednisolone (60 mg par jour).

Le paracétamol / codéine et le mouthwash (0,12 chlorexidine) ont été prescrits après l'opération.

La procédure a été réalisée sous anesthésie locale Ubistesine® avec vasoconstricteur 1/200000.

Une décontamination à la Bétadine® a été effectuée par voie intra et extra-buccale. Une incision est faite avec la lame du scalpel 15 C, supracrestale puis intra-sulculaire pour se terminer avec deux incisions à libération buccale. Un lambeau de tissus complet a été levé, puis le lambeau est relâché après les incisions périostées. Le lambeau est disséqué à l'aide d'une paire de ciseaux Metzenbaum®.

La maille / coque en zircone personnalisée est positionnée. Le forage a été effectué à travers les trous de l'enveloppe existante. Les deux vis d'ostéosynthèse sont partiellement vissées, puis les biomatériaux allogéniques sont placés sous la coque. Les vis ont été serrées pour assurer la stabilité du biomatériau. Des sutures ont été réalisées, bord à bord, sans tension.

## Revendications

1. Dispositif de régénération osseuse guidée, destiné à la reconstruction d'un défaut osseux buccal, composé de dioxyde de zirconium et ayant une forme recouvrant ledit défaut osseux buccal, ledit dispositif comprenant au moins une fenêtre aménagée dans la paroi de celui-ci et destinée à l'insertion et à la condensation d'un biomatériau.

2. Dispositif selon la revendication 1, **caractérisé en ce qu'**il contient en outre au moins un autre constituant choisi parmi l'oxyde d'yttrium, l'oxyde d'hafnium et l'oxyde d'aluminium.

3. Dispositif selon l'une des revendications 1 ou 2, **caractérisé en ce qu'**il possède une forme choisie parmi une coquille, une plaque et un filet.

4. Dispositif selon l'une quelconque des revendications précédentes, comprenant au moins une perforation de stabilisation dudit dispositif, ladite perforation étant destinée à recevoir une vis d'ostéosynthèse.

5. Dispositif selon l'une quelconque des revendications précédentes, ayant une épaisseur comprise entre 0,6 mm et 1,8 mm.

6. Procédé de fabrication d'un dispositif tel que défini dans l'une quelconque des revendications 1 à 5, comprenant une étape de construction dudit dispositif en fonction d'une représentation 3D obtenue par une technique d'imagerie dento-maxillaire du défaut osseux.

7. Procédé selon la revendication 6, dans lequel ladite représentation 3D dudit défaut osseux est schématisée numériquement sur un logiciel adapté pour quantifier la substance osseuse à régénérer.

8. Procédé selon l'une des revendications 6 ou 7, dans lequel ladite technique d'imagerie médicale est une technique de tomographie volumique à faisceaux coniques (Cône beam).

9. Procédé selon l'une quelconque des revendications 6 à 8, dans lequel ladite schématisation numérique permet d'obtenir un schéma, sur lequel une couche supplémentaire optionnelle de 1mm d'épaisseur est ajoutée.

## Patentansprüche

1. Vorrichtung zur geführten Knochenregeneration, die für die Rekonstruktion eines oralen Knochendefekts bestimmt ist, aus Zirkoniumdioxid besteht und eine den oralen Knochendefekt abdeckende Form aufweist, wobei die Vorrichtung mindestens ein in ihrer Wandung angeordnetes Fenster aufweist, das für das Einbringen und Kondensieren eines Biomaterials bestimmt ist.

2. Vorrichtung nach Anspruch 1, **dadurch gekennzeichnet, daß** sie ferner mindestens einen weiteren Bestandteil, ausgewählt aus Yttriumoxid, Hafniumoxid und Aluminiumoxid, enthält.

3. Vorrichtung nach einem der Ansprüche 1 oder 2, **dadurch gekennzeichnet, daß** sie eine Form aufweist, die aus einer Schale, einer Platte und einem Netz ausgewählt ist.

4. Vorrichtung nach einem der vorhergehenden Ansprüche, umfassend mindestens eine Perforation zur Stabilisierung der Vorrichtung, wobei die Perforation zur Aufnahme einer Osteosyntheseschraube vorgesehen ist.

5. Vorrichtung nach einem der vorhergehenden Ansprüche, mit einem Dicken zwischen 0,6 mm und 1,8 mm.

6. Verfahren zur Herstellung einer Vorrichtung nach einem der Ansprüche 1 bis 5, umfassend einen Schritt der Konstruktion der Vorrichtung in Abhängigkeit von einer 3D-Darstellung, die durch eine dento-maxilläre Bildgebungstechnik des Knochendefekts erhalten wird.

7. Verfahren nach Anspruch 6, wobei die 3D-Darstellung des Knochendefekts digital auf einer Software abgebildet wird, die zur Quantifizierung der zu regenerierenden Knochensubstanz geeignet ist.

8. Verfahren nach einem der Ansprüche 6 oder 7, wobei die medizinische Bildgebungstechnik eine Kegelstrahl-Volumentomographietechnik ist.

9. Verfahren nach einem der Ansprüche 6 bis 8, wobei die numerische Schematisierung zu einem Muster führt, auf das eine optionale zusätzliche Schicht von 1 mm Dicke aufgebracht wird.

## Claims

1. Device for guided bone regeneration, intended for the reconstruction of an oral bone defect, composed of zirconium dioxide and having a shape covering said oral bone defect, said device comprising at least one window arranged in the wall thereof and intended for the insertion and condensation of a biomaterial.

2. Device according to claim 1, **characterised in that** it additionally contains at least one further constituent selected from yttrium oxide, hafnium oxide and aluminium oxide

3. Device according to any of claims 1 or 2, **characterised in that** it has a shape selected from a shell, a plate and a net.

4. Device according to any one of the preceding claims, comprising at least one perforation for stabilising said device, said perforation being intended to receive an osteosynthesis screw.

5. Device according to any of the preceding claims, having a thickness of between 0.6 mm and 1.8 mm.

6. Method of manufacturing a device as defined in any one of claims 1 to 5, comprising a step of constructing said device according to a 3D representation obtained by a dento-maxillary imaging technique of the bone defect.

7. Method according to claim 6, wherein said 3D representation of said bone defect is digitally schematized on software adapted to quantify the bone substance to be regenerated.

8. Method according to any of claims 6 or 7, wherein said medical imaging technique is a Cone Beam Volumetric Tomography technique.

9. Method according to any one of claims 6 to 8, wherein said digital patterning results in a pattern, to which an optional additional layer of 1mm thickness is added.
